(19) Europäisches Patentamt
European Patent Office
Office européen des brevets

(11) Veröffentlichungsnummer: **0 062 003**
**B1**

(12) # EUROPÄISCHE PATENTSCHRIFT

(45) Veröffentlichungstag der Patentschrift: **27.12.85**

(51) Int. Cl.⁴: **C 07 C 149/23**, C 07 D 307/86, A 01 N 47/24

(21) Anmeldenummer: **82810130.3**

(22) Anmeldetag: **22.03.82**

(54) **Neue carboxylierte Oximcarbamate, Verfahren zu ihrer Herstellung, und ihre Verwendung in der Schädlingsbekämpfung.**

(30) Priorität: **26.03.81 CH 2073/81**
**08.01.82 CH 100/82**

(43) Veröffentlichungstag der Anmeldung:
**06.10.82 Patentblatt 82/40**

(45) Bekanntmachung des Hinweises auf die Patenterteilung:
**27.12.85 Patentblatt 85/52**

(84) Benannte Vertragsstaaten:
**AT BE CH DE FR GB IT LI NL**

(56) Entgegenhaltungen:
**EP - A - 0 022 498**
**DE - A - 2 111 459**

(73) Patentinhaber: **CIBA-GEIGY AG, Klybeckstrasse 141, CH-4002 Basel (CH)**

(72) Erfinder: **Drabek, Jozef, Dr., Benkenstrasse 12, CH-4104 Oberwil (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 062 003 B1

BUNDESDRUCKEREI BERLIN

**Beschreibung**

Die vorliegende Erfindung betrifft carboxylierte Oximcarbamate, Verfahren zu ihrer Herstellung und ihre Verwendung in der Schädlingsbekämpfung. Die carboxylierten Carbamate haben die Formel

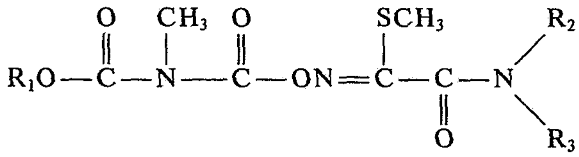

(I)

worin $R_1$ einen gegebenenfalls durch Halogenatom, Dioxolanyl-, Dioxanyl-, Alkyl-, Alkenyl-, Alkinyl-, Alkoxy- oder Alkylthiogruppen substituierten Phenyl-, Naphthyl- oder Dihydrobenzofuranylrest und $R_2$ und $R_3$ unabhängig voneinander Wasserstoff oder $C_1$—$C_4$-Alkyl bedeuten.

Als Halogenatome kommen Fluor-, Chlor-, Brom- oder Jodatome, insbesondere Chloratom in Betracht. Im Falle einer Substitution der Reste $R_1$ weisen diese vorzugsweise 1 bis 3 der erwähnten Substituenten auf. Die Alkyl-, Alkenyl-, Alkinyl-, Alkoxy- und Alkylthiogruppen können geradkettig oder verzweigt, unsubstituiert oder durch Halogen und/oder Alkoxy substituiert sein und haben in der Kette vorzugsweise 1 bis 6 resp. 2 bis 6 Kohlenstoffatome. Beispiele solcher Gruppen sind u. a.: Methyl, Methoxy, Methylthio, Äthyl, Äthoxy, 1-Methoxy-2-chloräthoxy, 1-Methoxymethyl-äthoxy, Äthylthio, Propyl, Propoxy, Isopropyl, Isopropoxy, n-, i-, sek.-, tert.-Butyl, n-Pentyl, n-Hexyl, Vinyl, Allyl, Äthinyl und Propargyl.

Die Alkylgruppen bei $R_2$ und $R_3$ sind Methyl, Äthyl, Propyl, Isopropyl, n-, i-, sek.- und tert.-Butyl, insbesondere aber Methyl. Unter Dioxolanyl- und Dioxanylgruppen sind Gruppen der Formeln

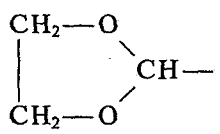

und

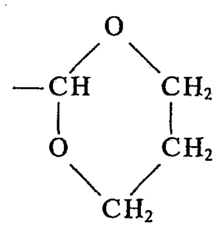

zu verstehen.

Besonders sind Verbindungen der Formel I, worin $R_1$ einen gegebenenfalls substituierten Phenyl-, Naphthyl- oder Dihydrobenzofuranylrest und $R_2$ und $R_3$ unabhängig voneinander Wasserstoff oder Methyl bedeuten. Insbesondere bevorzugt sind aber Verbindungen der Formel I, worin $R_1$ einen gegebenenfalls substituierten Phenyl-, Naphthyl- oder Dihydrobenzofuranylrest und $R_2$ und $R_3$ je Methyl bedeutet. Speziell bevorzugt sind Verbindungen der Formel I, worin $R_1$ unsubstituiertes Phenyl, Naphtyl oder Dihydrobenzofuranyl oder durch 1 bis 3 Fluor-, Chlor-, Brom- oder Jodatome oder Dioxolanyl-, Dioxanyl-, Alkyl-, Alkenyl-, Alkinyl-, Alkoxy- oder Alkylthiogruppen substituiertes Phenyl, Naphthyl oder Dihydrobenzofuranyl und $R_2$ und $R_3$ unabhängig voneinander Wasserstoff oder Methyl bedeuten.

Ganz besonders bevorzugt sind Verbindungen der Formel I, worin $R_1$ Phenyl, 4-Chlorphenyl, 2-(1-Methoxymethyläthoxy)-phenyl, 2-Isopropoxyphenyl, 2-(1-Methoxy-2-chloräthoxy)-phenyl, Naphthyl oder 2,2-Dimethyl-2,3-dihydrobenzofuranyl und $R_2$ und $R_3$ je Methyl bedeuten.

Davon speziell zu erwähnen sind Verbindungen der Formel I, worin $R_1$ unsubstituiertes Phenyl, 4-Chlorphenyl, Naphthyl oder 2,2-Dimethyl-2,3-dihydrobenzofuranyl und $R_2$ und $R_3$ je Methyl bedeuten.

Die Verbindungen der Formel I können nach an sich bekannten Methoden z. B. wie folgt hergestellt werden:

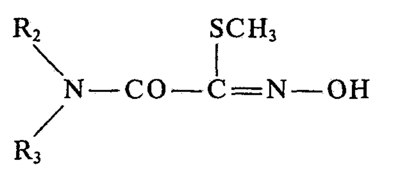

+

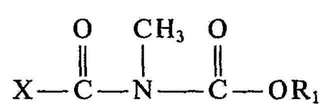

(II) (III)

In den Formeln II und III haben $R_1$, $R_2$ und $R_3$ die für die Formel I angegebene Bedeutung und X steht für ein Halogenatom, insbesondere für Fluor oder Chlor.

Das Verfahren wird bei einer Reaktionstemperatur zwischen $-50°$ und $+130°$, vorzugsweise zwischen $-10°$ und $+100°$, bei normalem oder leicht erhöhtem Druck und in Gegenwart einer Base und gegebenenfalls eines gegenüber den Reaktionsteilnehmern inerten Lösungs- oder Verdünnungsmittels vorgenommen.

Als geeignete Basen für dieses Verfahren kommen insbesondere tertiäre Amine, wie Trialkylamine,

Pyridine und Dialkylaniline ferner Hydroxide, Oxide, Carbonate und Bicarbonate von Alkali- und Erdalkalimetallen sowie Alkalimetallalkoholate wie z. B. Kalium-tert.butylat oder Natrium-methylat in Betracht.

Als Lösungs- oder Verdünnungsmittel eignen sich z. B. Äther und ätherartige Verbindungen wie Diäthyläther, Di-iso-propyläther, Dioxan, Tetrahydrofuran; aliphatische und aromatische Kohlenwasserstoffe, insbesondere Benzol, Toluol, Xylole; und Ketone wie Aceton, Methyläthylketon und Cyclohexanon.

Die Ausgangsstoffe der Formeln II und III sind bekannt bzw. können analog bekannten Methoden hergestellt werden.

Die Verbindungen der Formel I eignen sich zur Bekämpfung von Schädlingen an Tieren und Pflanzen.

Insbesondere eignen sich die Verbindungen der Formel I zur Bekämpfung von Insekten z. B. der Ordnung Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera und von phytopathogenen Milben und Zecken der Ordnung Acarina.

Vor allem eignen sich Verbindungene der Formel I zur Bekämpfung von pflanzenschädigenden Insekten, insbesondere pflanzenschädigenden Fraßinsekten, in Zier- und Nutzpflanzen, insbesondere in Baumwollkulturen (z. B. Spodoptera littoralis und Heliothis virescens) und Gemüsekulturen (z. B. Leptinotarsa decemlineata und Myzus persicae).

In diesem Zusammenhang ist hervorzuheben, daß die genannten Verbindungen sich sowohl durch eine stark ausgeprägte systemische als auch Kontakt-Wirkung gegen saugende Insekten insbesondere gegen saugende Insekten der Ordnung Homoptera und vor allem gegen Insekten der Familie Aphididae (wie z. B. Aphis fabae, Aphis craccivora und Myzus persicae), welche sich mit bekannten Mitteln nur schwierig bekämpfen lassen, auszeichnen.

Wirkstoffe der Formel I zeigen auch eine sehr günstige Wirkung gegen Fliegen, wie z. B. Musca domestica und Mückenlarven. Ferner zeichnen sie sich durch eine breite ovizide und ovolarvizide Wirkung aus und besitzen eine wertvolle Wirkung gegen ektoparasitäre Milben und Zecken z. B. der Familien Ixodidae, Argasidae und Dermanyssidae.

Die Verbindungen der Formel I werden in unveränderter Form oder vorzugsweise zusammen mit den in der Formulierungstechnik üblichen Hilfsmitteln eingesetzt und werden daher z. B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z. B. polymeren Stoffen in bekannter Weise verarbeitet. Die Anwendungsverfahren wie Versprühen, Vernebeln, Verstäuben, Verstreuen oder Gießen werden gleich wie die Art der Mittel den angestrebten Zielen und den gegebenen Verhältnissen entsprechend gewählt.

Die Formulierungen, d. h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z. B. durch inniges Vermischen und/oder Vermahlen der Wirkstoffe mit Streckmitteln, wie z. B. Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z. B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Äther und Ester, wie Äthanol Äthylenglykol, Äthylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle wie epoxidiertes Kokosnußöl oder Sojaöl; oder Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen, wie z. B. Bimsstein, Ziegelbruch, Sepiolit oder Bentonit, als nicht sorptive Trägermaterialien z. B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$—$C_{22}$), wie z. B. die Na- oder K-Salze der Öl- oder Stearinsäure, oder von natürlichen Fettsäuregemischen, die z. B. aus Kokosnuß- oder Tallöl gewonnen werden können. Ferner sind auch die Fettsäure-methyl-taurinsalze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschließt, z. B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Äthylenoxid-Addukten. Die sulfonierten Benzimidazolderivate enthalten vorzugsweise 2 Sulfonsäuregruppen und einen Fettsäurerest mit 8—22 C-Atomen. Alkylarylsulfonate sind z. B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z. B. Salze des Phosphorsäureesters eines p-Nonylphenol-(4—14)-Äthylenoxid-Adduktes in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Äthylenglykoläthergruppen und 10 bis 100 Propylenglykoläthergruppen enthaltenden Polyäthylenoxid-Addukte an Polypropylenglykol, Äthylendiaminpolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Äthylenglykoleinheiten.

Als Beispiele nichtionischer Tenside seien Nonylphenolpolyäthoxyäthanole, Ricinusölpolyglycoläther, Polypropylen-Polyäthylenoxid-Addukte, Tributylphenoxypolyäthoxyäthanol, Polyäthylenglykol und Octylphenoxyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Äthylsulfate vor, z. B. das Stearyltrimethylammoniumchlorid oder das Benzyldi(2-chloräthyl)-äthylammoniumbromid.

Die in der Formulierungstechnik gebräuchlichen Tenside sind u. a. in folgender Publikation beschrieben:

»Mc Cutcheon's Detergents and Emulsifiers Annual« MC Publishing Corp., Ridgewood, New Jersey, 1979.

Die pestiziden Zubereitungen enthalten in der Regel 0,1 bis 99%, insbesondere 0,1 bis 95%, Wirkstoff der Formel I, 1 bis 99,9% eines festen oder flüssigen Zusatzstoffes und 0 bis 25%, insbesondere 0,1 bis 25%, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Formulierungsbeispiele für flüssige Wirkstoffe der Formel I
(% = Gewichtsprozent)

| 1. Emulsions-Konzentrate | a) | b) | c) |
|---|---|---|---|
| Wirkstoff | 20% | 40% | 50% |
| Ca-Dodecylbenzolsulfonat | 5% | 8% | 5,8% |
| Ricinusöl-polyäthylenglykoläther (36 Mol AeO) | 5% | — | — |
| Tributylphenyl-polyäthylenglykoläther (30 Mol AeO) | — | 12% | 4,2% |
| Cyclohexanon | — | 15% | 20% |
| Xylolgemisch | 70% | 25% | 20% |

Aus solchen Konzentraten können durch Verdünnen mit Wasser Emulsionen jeder gewünschten Konzentration hergestellt werden.

| 2. Lösungen | a) | b) | c) | d) |
|---|---|---|---|---|
| Wirkstoff | 80% | 10% | 5% | 95% |
| Äthylenglykol-monomethyl-äther | 20% | – | – | – |
| Polyäthylenglykol MG 400 | – | 70% | – | – |
| N-Methyl-2-pyrrolidon | – | 20% | – | – |
| Epoxidiertes Kokosnußöl | – | – | 1% | 5% |
| Benzin (Siedegrenzen 160–190°C) | – | – | 94% | – |

Die Lösungen sind zur Anwendung in Form kleinster Tropfen geeignet.

| 3. Granulate | a) | b) |
|---|---|---|
| Wirkstoff | 5% | 10% |
| Kaolin | 94% | |
| Hochdisperse Kieselsäure | 1% | – |
| Attapulgit | – | 90% |

Der Wirkstoff wird in Methylenchlorid gelöst, auf den Träger aufgesprüht und das Lösungsmittel anschließend im Vakuum abgedampft.

| 4. Stäubemittel | a) | b) |
|---|---|---|
| Wirkstoff | 2% | 5% |
| Hochdisperse Kieselsäure | 1% | 5% |
| Talkum | 97% | – |
| Kaolin | – | 90% |

Durch inniges Vermischen der Trägerstoffe mit dem Wirkstoff erhält man gebrauchsfertige Stäubemittel.

Formulierungsbeispiele für feste Wirkstoffe der Formel I
(% = Gewichtsprozent)

| 5. Spritzpulver | a) | b) |
|---|---|---|
| Wirkstoff | 20% | 60% |
| Na-Ligninsulfonat | 5% | 5% |
| Na-Laurylsulfat | 3% | – |
| Na-Diisobutylnaphthalinsulfonat | – | 6% |
| Octylphenolpolyäthylenglykoläther (7–8 Mol AeO) | – | 2% |
| Hochdisperse Kieselsäure | 5% | 27% |
| Kaolin | 67% | – |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

6. Emulsions-Konzentrat

| | |
|---|---|
| Wirkstoff | 10% |
| Octylphenolpolyäthylenglykoläther (4–5 Mol AeO) | 3% |
| Ca-Dodecylbenzolsulfonat | 3% |
| Ricinusölpolyglykoläther (36 Mol AeO) | 4% |
| Cyclohexanon | 30% |
| Xylolgemisch | 50% |

Aus diesem Konzentrat können durch Verdünnen mit Wasser Emulsionen jeder gewünschten Konzentration hergestellt werden.

| 7. Stäubemittel | a) | b) |
|---|---|---|
| Wirkstoff | 5% | 8% |
| Talkum | 95% | – |
| Kaolin | – | 92% |

Man erhält anwendungsfertige Stäubemittel, indem der Wirkstoff mit dem Träger vermischt und auf einer geeigneten Mühle vermahlen wird.

8. Extruder Granulat

| | |
|---|---|
| Wirkstoff | 10% |
| Na-Ligninsulfonat | 2% |
| Carboxymethylcellulose | 1% |
| Kaolin | 87% |

Der Wirkstoff wird mit den Zusatzstoffen vermischt, vermahlen und mit Wasser angefeuchtet. Dieses Gemisch wird extrudiert und anschließend im Luftstrom getrocknet.

9. Umhüllungs-Granulat

| | |
|---|---|
| Wirkstoff | 3% |
| Polyäthylenglykol (MG 200) | 3% |
| Kaolin | 94% |

Der fein gemahlene Wirkstoff wird in einem Mischer auf das mit Polyäthylenglykol angefeuchtete Kaolin gleichmäßig aufgetragen. Auf diese Weise erhält man staubfreie Umhüllungs-Granulate.

10. Suspensions-Konzentrat

| | |
|---|---|
| Wirkstoff | 40% |
| Äthylenglykol | 10% |
| Nonylphenolpolyäthylenglykoläther (15 Mol AeO) | 6% |
| Na-Ligninsulfonat | 10% |
| Carboxymethylcellulose | 1% |
| 37%ige wäßrige Formaldehyd-Lösung | 0,2% |
| Silikonöl in Form einer 75%igen wäßrigen Emulsion | 0,8% |
| Wasser | 32% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 1

Herstellung der Verbindung der Formel

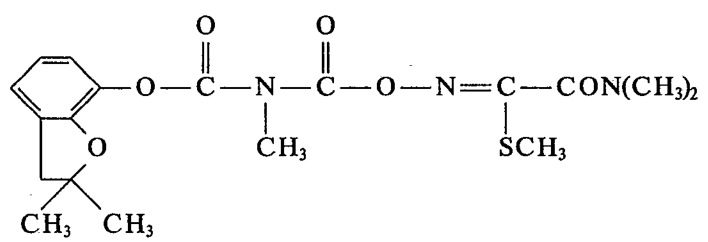

Zu einer Lösung von 4,05 g N,N-Dimethyl-2-oximino-2-(methylthio)-acetamid in 100 ml Toluol/Acetonitril (1 : 1), 5,5 ml Triäthylamin und 0,2 g 4-Dimethylaminopyridin werden bei 0 bis 10°C 2,28 g 2,2-Dimethyl-2,3-dihydrobenzofuranyl-N-chlorcarbonyl-N-methylcarbamat, in 50 ml Chloroform gelöst, zugetropft. Das Reaktionsgemisch wird 12 Stunden bei 20°C und 5 Stunden am Rückfluß gerührt. Nach dem Einengen wird der Rückstand in Toluol aufgenommen. Die Toluollösung wird nach dem Abnutschen zweimal mit 100 ml Wasser, je einmal mit 100 ml 1N-Salzsäure- und 100 ml 1N-Bikarbonatlösung gewaschen, über Natriumsulfat getrocknet und eingeengt.
Man erhält die Titelverbindung mit einer Refraktion von $n_D^{50°} = 1,5382$.
Auf analoge Weise werden auch folgende Verbindungen hergestellt:

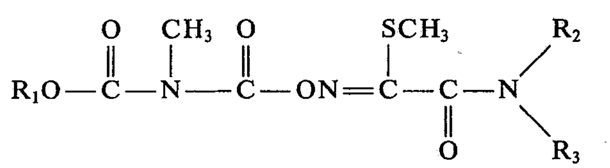

| Nr. | $R_1$ | $R_2$ | $R_3$ | Physikalische Daten |
|---|---|---|---|---|
| 2 | 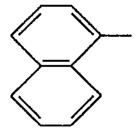 | $CH_3$ | $CH_3$ | $n_D^{50°} = 1,5933$ |

Fortsetzung

| Nr. | $R_1$ | $R_2$ | $R_3$ | Physikalische Daten |
|---|---|---|---|---|
| 3 | 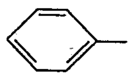 | $CH_3$ | $CH_3$ | $n_D^{20°} = 1{,}5408$ |
| 4 | 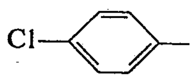 | $CH_3$ | $CH_3$ | $n_D^{20°} = 1{,}5570$ |
| 5 | 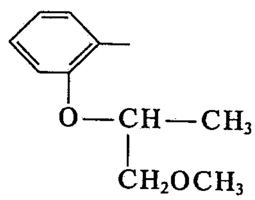 | $CH_3$ | $CH_3$ | $n_D^{20°} = 1{,}5376$ |
| 6 | 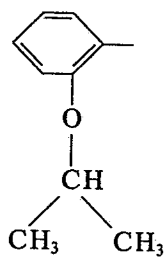 | $CH_3$ | $CH_3$ | $n_D^{20°} = 1{,}5408$ |
| 7 | 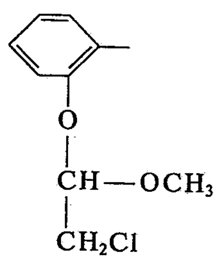 | $CH_3$ | $CH_3$ | $n_D^{50°} = 1{,}5477$ |
| 8 | 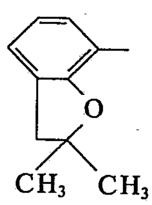 | H | H | Smp.: 150–151°C |
| 9 | 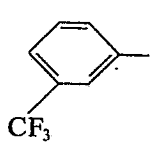 | $CH_3$ | $CH_3$ | $n_D^{50°} = 1{,}5142$ |
| 10 | 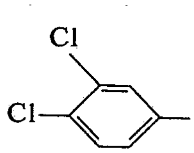 | $CH_3$ | $CH_3$ | Smp.: 136–138°C |

Beispiel 2

Insektizide systemische Wirkung: Aphis craccivora

Bewurzelte Bohnenpflanzen werden in Töpfe, welche 600 ccm Erde enthalten, verpflanzt. Anschließend werden 50 ml einer Versuchslösung, enthaltend 25 ppm, 5 ppm bzw. 1 ppm der zu prüfenden Verbindung direkt auf die Erde gegossen.

Nach 24 Stunden werden auf die oberirdischen Pflanzenteile Blattläuse (Aphis craccivora) gesetzt und die Pflanzen mit einem unten zugeschnürten Plastikzylinder überstülpt, um die Läuse vor einer eventuellen Kontakt- oder Gaswirkung der Testsubstanz zu schützen.

Die Auswertung der erzielten Abtötung erfolgt 48 Stunden nach Versuchsbeginn. Pro Konzentrationsdosis Testsubstanz werden zwei Pflanzen, je eine in einem separaten Topf, verwendet. Der Versuch wird bei 25°C und 70% relativer Luftfeuchtigkeit durchgeführt.

Die Verbindungen gemäß Beispiel 1 haben die in der folgenden Tabelle angegebene Wirkung gegen Aphis craccivora.

Biologische Versuchsergebnisse

In der folgenden Tabelle sind Versuchsergebnisse auf der Basis des vorstehenden Beispiels aufgeführt, und zwar mit folgendem Bewertungsindex in bezug auf die prozentuale Abtötung der Schädlinge:

A: 70–100% Abtötung bei 1 ppm Wirkstoffkonzentration
B: 70–100% Abtötung bei 5 ppm Wirkstoffkonzentration
C: 70–100% Abtötung bei 25 ppm Wirkstoffkonzentration

| Verbindung Nr. | Wirksamkeit gegen Aphis craccivora |
|---|---|
| 1 | A |
| 2 | B |
| 3 | B |
| 4 | A |
| 5 | A |
| 6 | A |
| 7 | B |
| 8 | C |
| 9 | B |
| 10 | A |

**Patentansprüche für die Vertragsstaaten: BE, CH, DE, FR, GB, IT, LI, NL**

1. Ein carboxyliertes Oximcarbonat der Formel

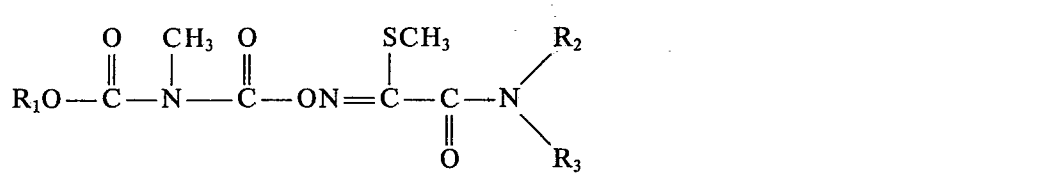

(I)

worin $R_1$ einen gegebenenfalls durch Halogenatome, Dioxolanyl-, Dioxanyl-, Alkyl-, Alkenyl-, Alkinyl-,

Alkoxy- oder Alkylthiogruppen substituierten Phenyl-, Naphthyl- oder Dihydrobenzofuranylrest und $R_2$ und $R_3$ unabhängig voneinander Wasserstoff und $C_1$—$C_4$-Alkyl bedeuten.

2. Eine Verbindung gemäß Anspruch 1, worin $R_2$ und $R_3$ unabhängig voneinander Wasserstoff oder Methyl bedeuten.

3. Eine Verbindung gemäß einem der Ansprüche 1 oder 2, worin $R_2$ und $R_3$ je Methyl bedeuten.

4. Eine Verbindung gemäß einem der Ansprüche 1 bis 3, worin $R_1$ unsubstituiertes Phenyl, Naphthyl oder Dihydrobenzofuranyl oder durch 1 bis 3 Fluor-, Chlor-, Brom- oder Jodatome oder Dioxolanyl-, Dioxanyl-, Alkyl-, Alkenyl-, Alkinyl-, Alkoxy- oder Alkylthiogruppen substituiertes Phenyl, Napththyl oder Dihydrobenzofuranyl bedeuten.

5. Eine Verbindung gemäß einem der Ansprüche 2 bis 4, worin $R_1$ Phenyl, 4-Chlorphenyl, 2-(1-Methoxymethyl-äthoxy)-phenyl, 2-Isopropoxyphenyl, 2-(1-Methoxy-2-chloräthoxy)-phenyl, 3-Trifluormethylphenyl, 3,4-Dichlorphenyl, Naphthyl oder 2,2-Dimethyl-2,3-dihydrobenzofuranyl bedeuten.

6. Eine Verbindung gemäß einem der Ansprüche 3 bis 5, worin $R_1$ Phenyl, 4-Chlorphenyl, Naphthyl oder 2,2-Dimethyl-2,3-dihydrobenzofuranyl bedeuten.

7. Die Verbindung gemäß Anspruch 6 der Formel

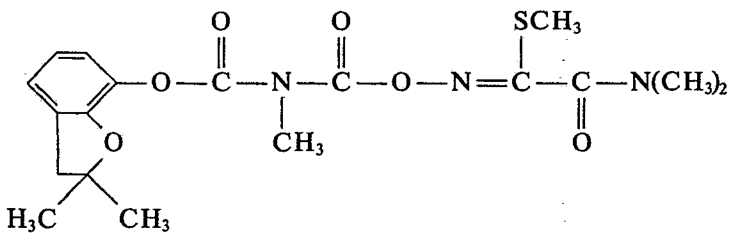

8. Die Verbindung gemäß Anspruch 6 der Formel

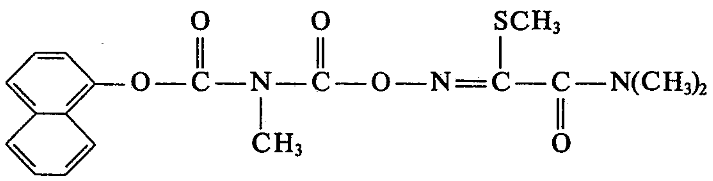

9. Die Verbindung gemäß Anspruch 6 der Formel

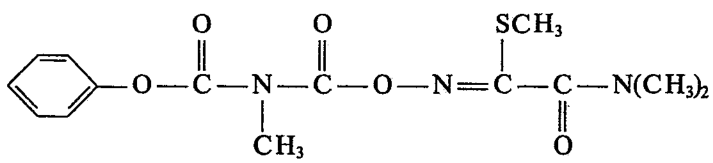

10. Die Verbindung gemäß Anspruch 6 der Formel

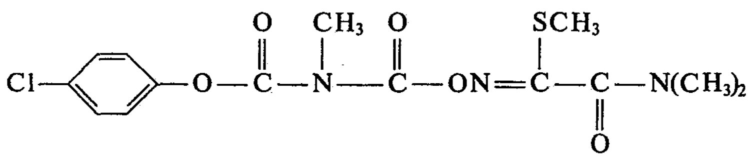

11. Die Verbindung gemäß Anspruch 5 der Formel

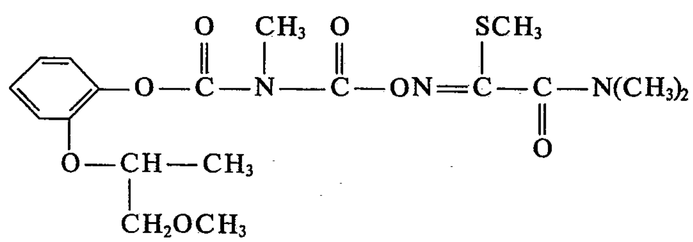

12. Die Verbindung gemäß Anspruch 5 der Formel

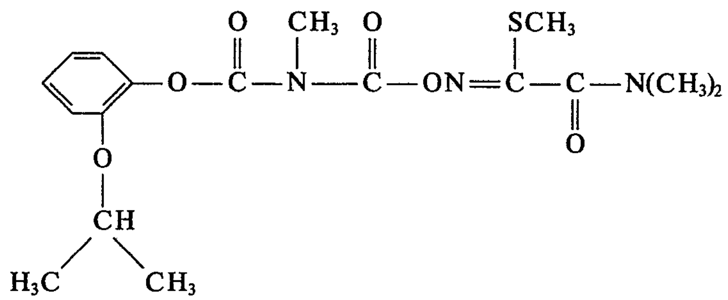

13. Die Verbindung gemäß Anspruch 5 der Formel

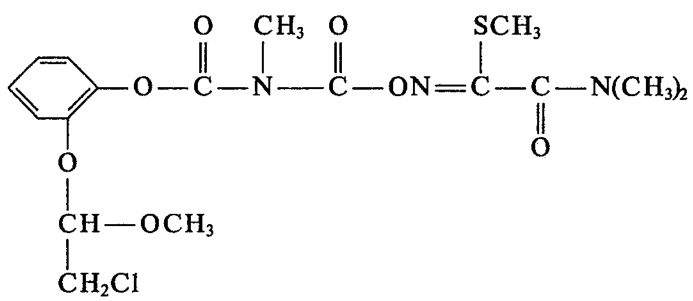

14. Die Verbindung gemäß Anspruch 5 der Formel

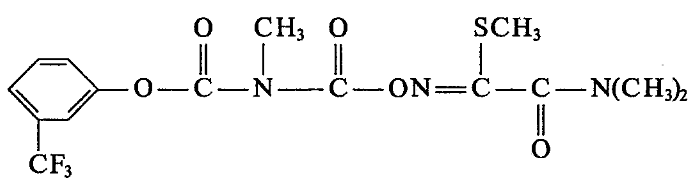

15. Die Verbindung gemäß Anspruch 5 der Formel

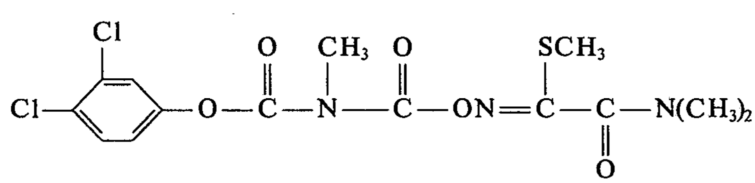

16. Die Verbindung gemäß Anspruch 5 der Formel

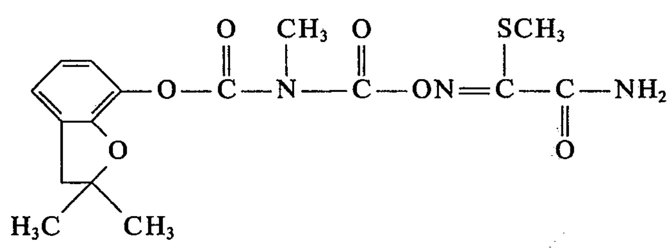

17. Verfahren zur Herstellung von Verbindungen gemäß Anspruch 1, dadurch gekennzeichnet, daß man in Gegenwart einer Base eine Verbindung der Formel

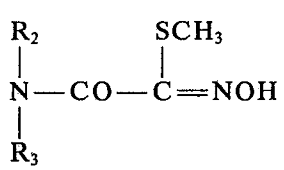

mit einer Verbindung der Formel

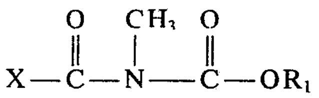

umsetzt, worin $R_1$, $R_2$ und $R_3$ die im Anspruch 1 angegebene Bedeutung haben und X für ein Halogenatom steht.

18. Ein Schädlingsbekämpfungsmittel, welches als aktive Komponente eine Verbindung gemäß Anspruch 1 enthält.

19. Die Verwendung einer Verbindung gemäß Anspruch 1 zur Bekämpfung von Schädlingen an Tieren, Pflanzen und im Boden.

20. Die Verwendung gemäß Anspruch 19 zur Bekämpfung von Insekten und Vertretern der Ordnung Akarina.

**Patentansprüche für den Vertragsstaat: AT**

1. Schädlingsbekämpfungsmittel, dadurch gekennzeichnet, daß es neben geeigneten Träger- und/oder anderen Zuschlagstoffen als aktive Komponente ein carboxyliertes Oximcarbamat der Formel

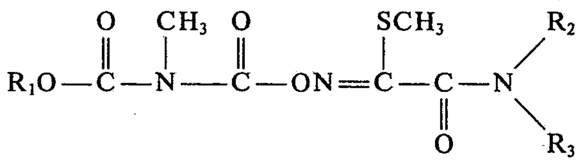 (I)

enthält, worin $R_1$ einen gegebenenfalls durch Halogenatome, Dioxolanyl-, Dioxanyl-, Alkyl-, Alkenyl-, Alkinyl-, Alkoxy- oder Alkylthiogruppen substituierten Phenyl-, Naphthyl- oder Dihydrobenzofuranylrest und $R_2$ und $R_3$ unabhängig voneinander Wasserstoff und $C_1$—$C_4$-Alkyl bedeuten.

2. Schädlingsbekämpfungsmittel gemäß Anspruch 1, dadurch gekennzeichnet, daß es als aktive Komponente eine Verbindung der Formel I enthält, worin $R_2$ und $R_3$ unabhängig voneinander Wasserstoff oder Methyl bedeuten.

3. Schädlingsbekämpfungsmittel gemäß einem der Ansprüche 1 oder 2, dadurch gekennzeichnet, daß es als aktive Komponente eine Verbindung der Formel I enthält, worin $R_2$ und $R_3$ je Methyl bedeuten.

4. Schädlingsbekämpfungsmittel gemäß einem der Ansprüche 1 bis 3, dadurch gekennzeichnet, daß es als aktive Komponente eine Verbindung der Formel I enthält, worin $R_1$ unsubstituiertes Phenyl, Naphthyl oder Dihydrobenzofuranyl oder durch 1 bis 3 Fluor-, Chlor-, Brom- oder Jodatome oder Dioxolanyl-, Dioxanyl-, Alkyl-, Alkenyl-, Alkinyl-, Alkoxy- oder Alkylthiogruppen substituiertes Phenyl, Napthyl oder Dihydrobenzofuranyl bedeutet.

5. Schädlingsbekämpfungsmittel gemäß einem der Ansprüche 2 bis 4, dadurch gekennzeichnet, daß es als aktive Komponente eine Verbindung der Formel I enthält, worin $R_1$ Phenyl, 4-Chlorphenyl, 2-(1-Methoxymethyl-äthoxy)-phenyl, 2-Isopropoxyphenyl, 2-(1-Methoxy-2-chlor-äthoxy)-phenyl, 3-Trifluormethylphenyl, 3,4-Dichlorphenyl, Naphthyl oder 2,2-Dimethyl-2,3-dihydrobenzofuranyl bedeutet.

6. Schädlingsbekämpfungsmittel gemäß einem der Ansprüche 3 bis 5, dadurch gekennzeichnet, daß es als aktive Komponente eine Verbindung der Formel I enthält, worin $R_1$ Phenyl, 4-Chlorphenyl, Naphthyl oder 2,2-Dimethyl-2,3-dihydrobenzofuranyl bedeutet.

7. Schädlingsbekämpfungsmittel gemäß Anspruch 6, dadurch gekennzeichnet, daß es als aktive Komponente die Verbindung der Formel

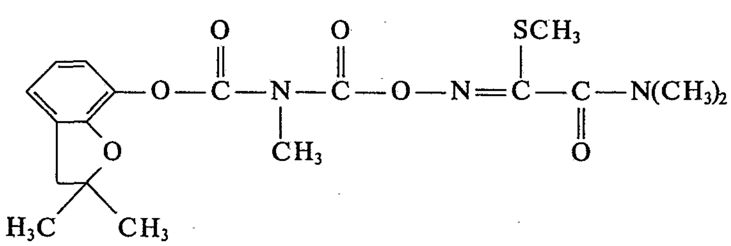

enthält.

8. Schädlingsbekämpfungsmittel gemäß Anspruch 6, dadurch gekennzeichnet, daß es als aktive Komponente die Verbindung der Formel

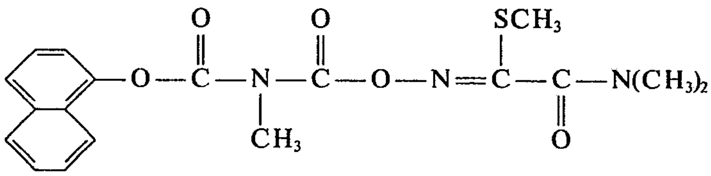

enthält.

9. Schädlingsbekämpfungsmittel gemäß Anspruch 6, dadurch gekennzeichnet, daß es als aktive Komponente die Verbindung der Formel

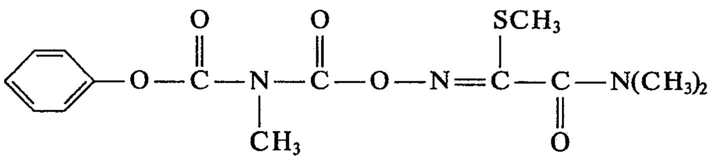

enthält.

10. Schädlingsbekämpfungsmittel gemäß Anspruch 6, dadurch gekennzeichnet, daß es als aktive Komponente die Verbindung der Formel

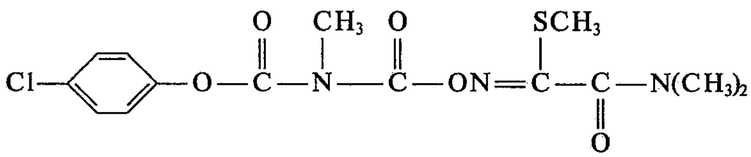

enthält.

11. Schädlingsbekämpfungsmittel gemäß Anspruch 5, dadurch gekennzeichnet, daß es als aktive Komponente die Verbindung der Formel

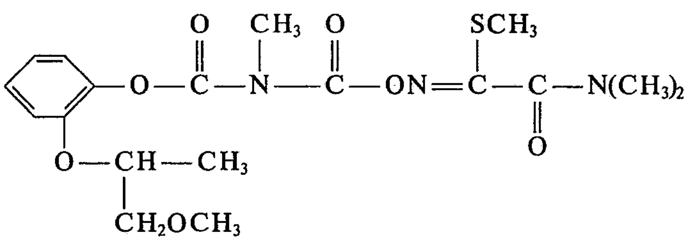

enthält.

12. Schädlingsbekämpfungsmittel gemäß Anspruch 5, dadurch gekennzeichnet, daß es als aktive Komponente die Verbindung der Formel

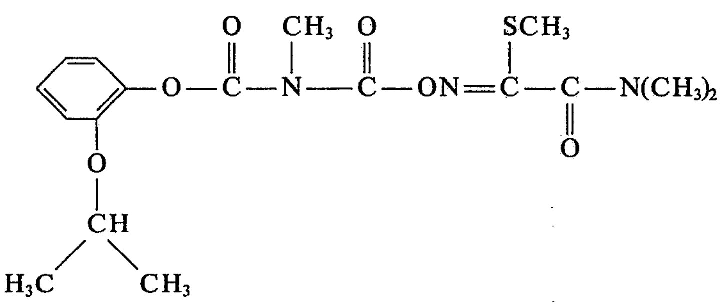

enthält.

13. Schädlingsbekämpfungsmittel gemäß Anspruch 5, dadurch gekennzeichnet, daß es als aktive Komponente die Verbindung der Formel

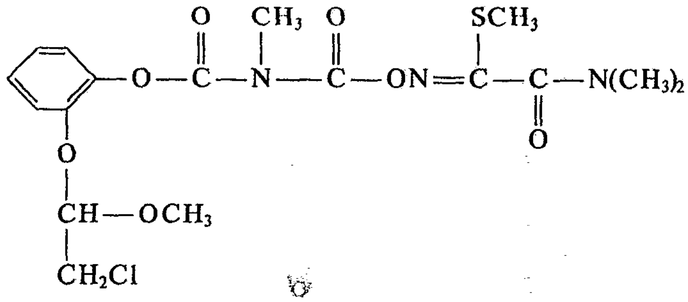

enthält.

14. Schädlingsbekämpfungsmittel gemäß Anspruch 5, dadurch gekennzeichnet, daß es als aktive Komponente die Verbindung der Formel

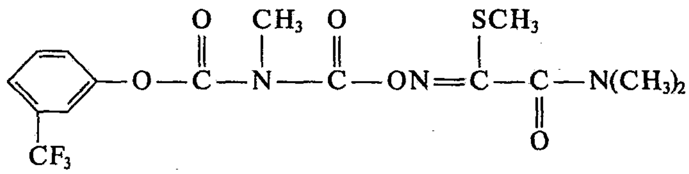

enthält.

15. Schädlingsbekämpfungsmittel gemäß Anspruch 5, dadurch gekennzeichnet, daß es als aktive Komponente die Verbindung der Formel

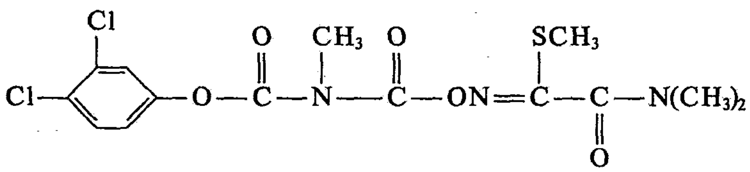

enthält.

16. Schädlingsbekämpfungsmittel gemäß Anspruch 5, dadurch gekennzeichnet, daß es als aktive Komponente die Verbindung der Formel

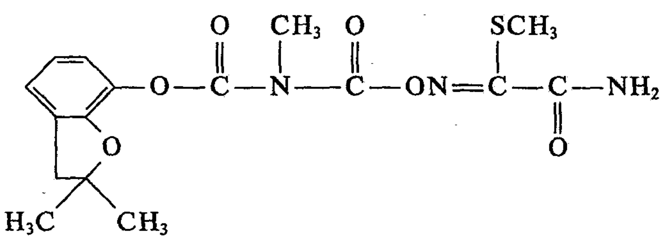

enthält.

17. Verfahren zur Herstellung von carboxylierten Carbamaten der Formel

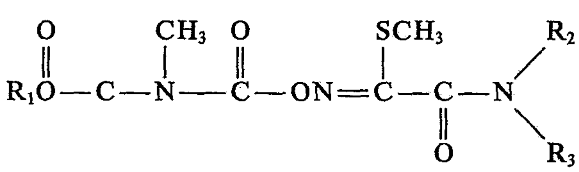

(I)

worin $R_1$ einen gegebenenfalls durch Halogenatome, Dioxolanyl-, Dioxanyl-, Alkyl-, Alkenyl-, Alkinyl-, Alkoxy- oder Alkylthiogruppen substituierten Phenyl-, Naphthyl- oder Dihydrobenzofuranylrest und $R_2$ und $R_3$ unabhängig voneinander Wasserstoff und $C_1$—$C_4$-Alkyl bedeuten, dadurch gekennzeichnet, daß man in Gegenwart einer Base eine Verbindung der Formel

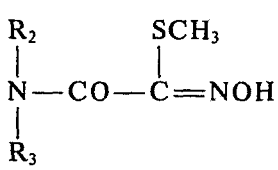

mit einer Verbindung der Formel

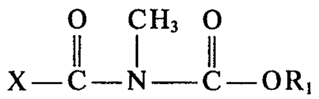

umsetzt, worin $R_1$, $R_2$ und $R_3$ die oben angegebene Bedeutung haben und X für ein Halogenatom steht.

**Claims for the Contracting States: DE, GB, FR, CH, LI, IT, NL, BE**

1. A carboxylated oxime carbamate of the formula

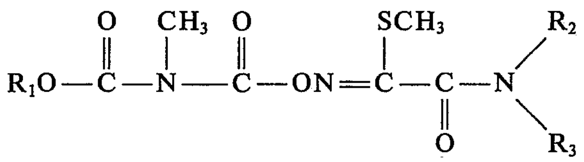

(I)

wherein $R_1$ is a phenyl, naphthyl or dihydrobenzofuranyl radical which is unsubstituted or substituted by halogen atoms, dioxolanyl, dioxanyl, alkyl, alkenyl, alkynyl, alkoxy or alkylthio groups and each of $R_2$ and $R_3$ independently is hydrogen or $C_1$—$C_4$ alkyl.

2. A compound according to claim 1, wherein each of $R_2$ and $R_3$ independently is hydrogen or methyl.

3. A compound according to either of claims 1 or 2, wherein each of $R_2$ and $R_3$ is methyl.

4. A compound according to any one of claims 1 to 3, wherein $R_1$ is an unsubstituted phenyl, naphthyl or dihydrobenzofuranyl radical or a phenyl, naphthyl or dihydrobenzofuranyl radical which is substituted by 1 to 3 fluorine, chlorine, bromine or iodine atoms or dioxolanyl, dioxanyl, alkyl, alkenyl, alkynyl, alkoxy or alkylthio groups.

5. A compound according to any one of claims 2 to 4, wherein $R_1$ is phenyl, 4-chlorophenyl, 2-(1-methoxymethylethoxy)phenyl, 2-isopropoxyphenyl, 2-(1-methoxy-2-chloroethoxy)phenyl, 3-trifluoromethylphenyl, 3,4-dichlorophenyl, naphthyl or 2,2-dimethyl-2,3-dihydrobenzofuranyl.

6. A compound according to any one of claims 3 to 5, wherein $R_1$ is phenyl, 4-chlorophenyl, naphthyl or 2,2-dimethyl-2,3-dihydrobenzofuranyl.

7. The compound according to claim 6 of the formula

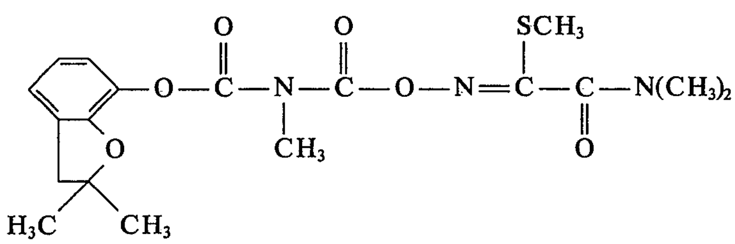

8. The compound according to claim 6 of the formula

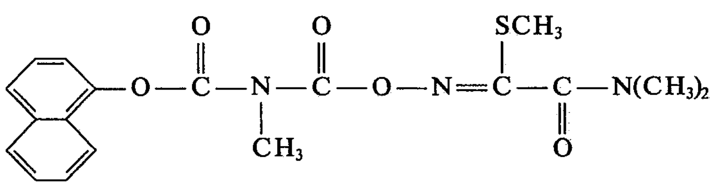

9. The compound according to claim 6 of the formula

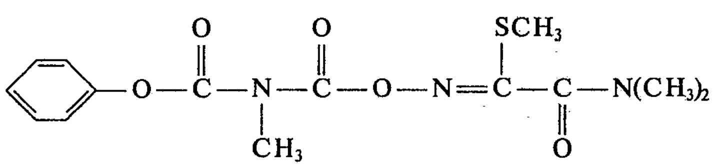

10. The compound according to claim 6 of the formula

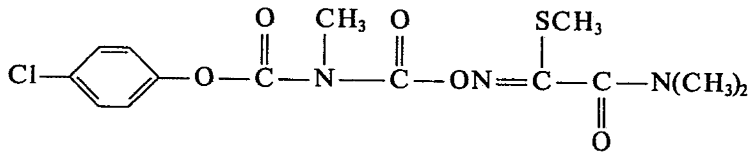

11. The compound according to claim 5 of the formula

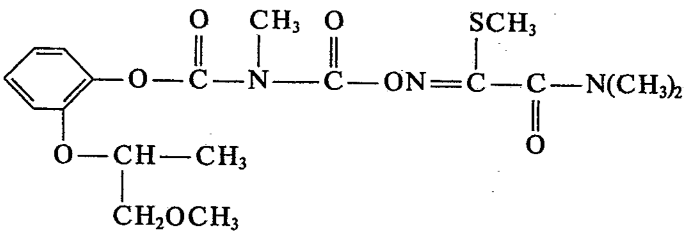

12. The compound according to claim 5 of the formula

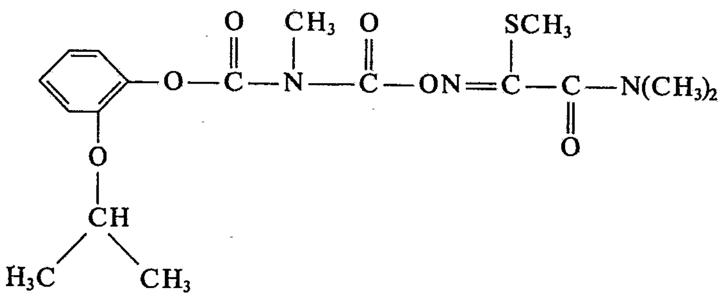

13. The compound according to claim 5 of the formula

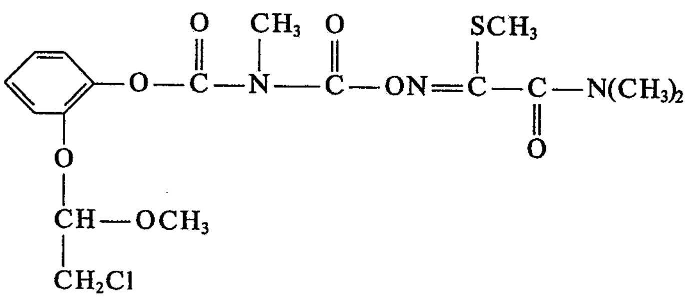

14. The compound according to claim 5 of the formula

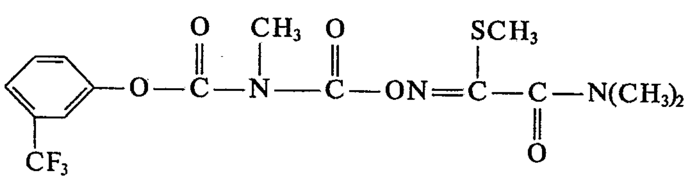

15. The compound according to claim 5 of the formula

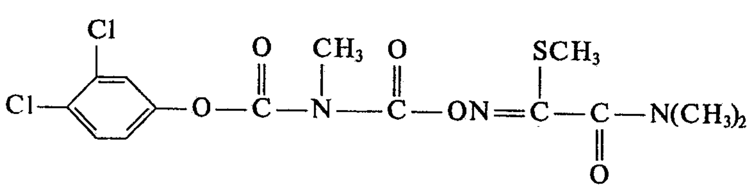

16. The compound according to claim 5 of the formula

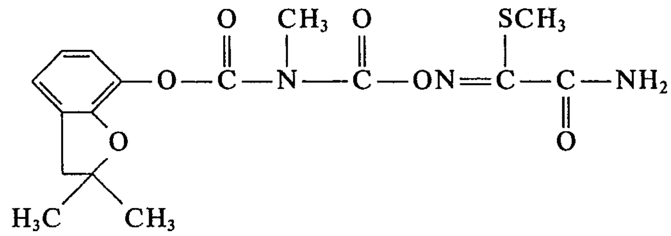

17. A process for the preparation of a compound according to claim 1, which process comprises reacting a compound of the formula

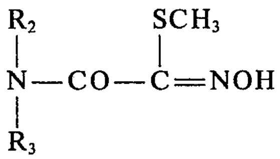

with a compound of the formula

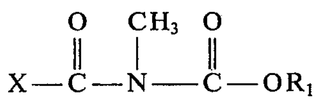

in which formulae $R_1$, $R_2$ and $R_3$ are as defined in claim 1 and X is halogen atom, in the presence of a base.

18. A pesticidal composition which contains as active ingredient a compound according to claim 1.

19. Use of a compound according to claim 1 for controlling pests of animals and plants and pests in the soil.

20. Use according to claim 19 for controlling insects and representatives of the order Acarina.

**Claims for the Contracting State: AT**

1. A pesticidal composition which contains as active ingredient a carboxylated oxime carbamate of the formula

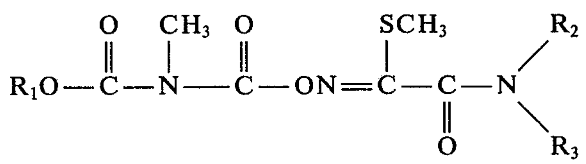

(I)

wherein $R_1$ is a phenyl, naphthyl or dihydrobenzofuranyl radical which is unsubstituted or substituted by halogen atoms, dioxolanyl, dioxanyl, alkyl, alkenyl, alkynyl, alkoxy or alkylthio groups and each of $R_2$ and $R_3$ independently is hydrogen or $C_1$—$C_4$ alkyl, togehter with suitable carriers and/or other adjuvants.

2. A pesticidal composition according to claim 1, which contains as active ingredient a compound of the formula I, wherein each of $R_2$ and $R_3$ independently is hydrogen or methyl.

3. A pesticidal composition according to either of claims 1 or 2, which contains as active ingredient a compound of the formula I, wherein each of $R_2$ and $R_3$ is methyl.

4. A pesticidal composition according to any one of claims 1 to 3, which contains as active ingredient a compound of the formula I, wherein $R_1$ is an unsubstituted phenyl, naphthyl or dihydrobenzofuranyl radical or a phenyl, naphthyl or dihydrobenzofuranyl radical which is substitited by 1 to 3 fluorine, chlorine, bromine or iodine atoms or dioxolanyl, dioxanyl, alkyl, alkenyl, alkynyl, alkoxy or alkylthio groups.

5. A pesticidal composition according to any one of claims 2 to 4, which contains as active ingredient a compound of the formula I, wherein $R_1$ is phenyl, 4-chlorophenyl, 2-(1-methoxymethylethoxy)phenyl, 2-isopropoxyphenyl, 2-(1-methoxy-2-chloroethoxy)phenyl, 3-trifluoromethylphenyl, 3,4-dichlorophenyl, naphthyl or 2,2-dimethyl-2,3-dihydrobenzofuranyl.

6. A pesticidal composition according to any one of claims 3 to 5, which contains as active ingredient a compound of the formula I, wherein $R_1$ is phenyl, 4-chlorophenyl, naphthyl or 2,3-dimethyl-2,3-dihydrobenzofuranyl.

7. A pesticidal composition according to claim 6, which contains as active ingredient the compound of the formula

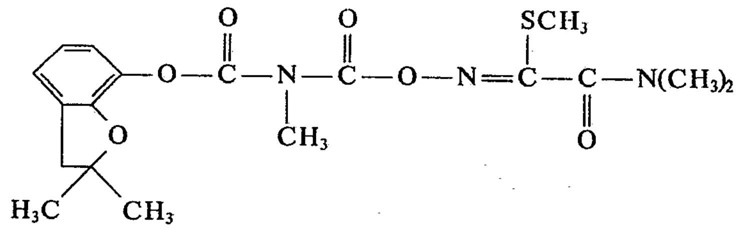

8. A pesticidal composition according to claim 6, which contains as active ingredient the compound of the formula

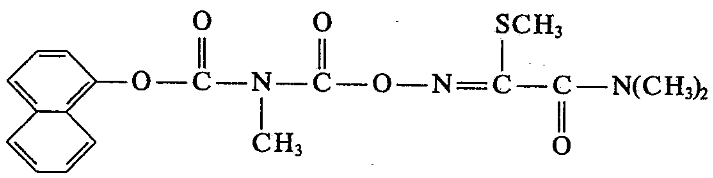

9. A pesticidal composition according to claim 6, which contains as active ingredient the compound of the formula

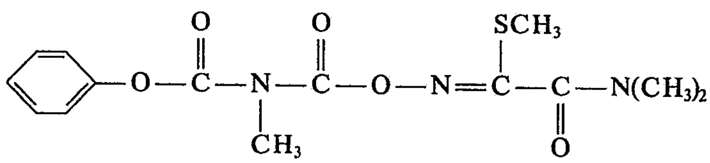

10. A pesticidal composition according to claim 6, which contains as active ingredient the compound of the formula

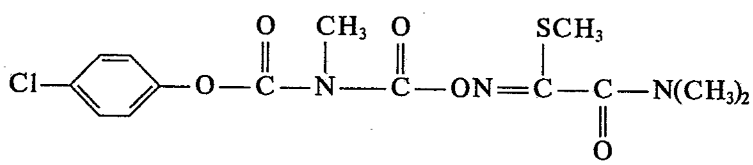

11. A pesticidal composition according to claim 5, which contains as active ingredient the compound of the formula

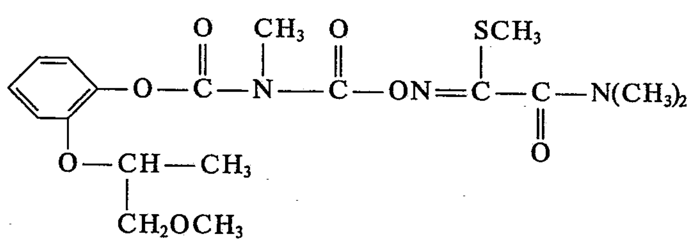

12. A pesticidal composition according to claim 5, which contains as active ingredient the compound of the formula

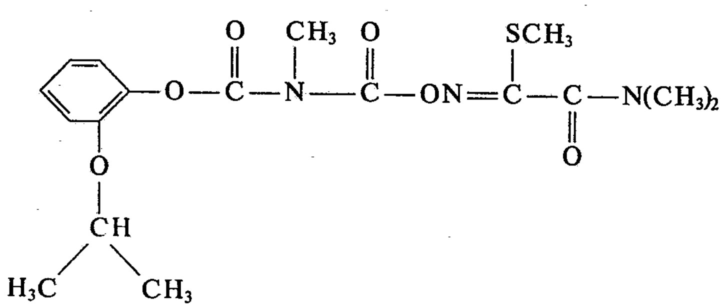

13. A pesticidal composition according to claim 5, which contains as active ingredient the compound of the formula

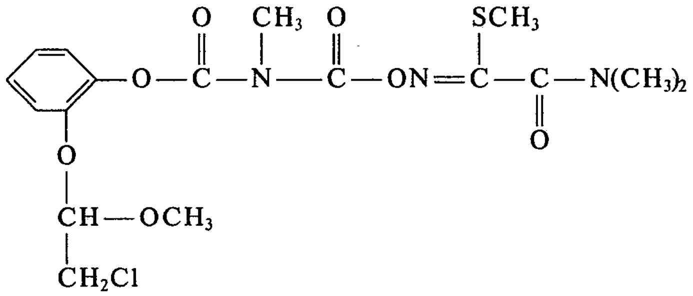

14. A pesticidal composition according to claim 5, which contains as active ingredient the compound of the formula

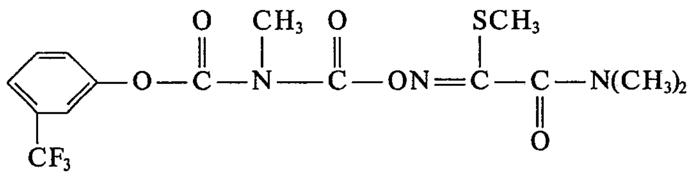

15. A pesticidal composition according to claim 5, which contains as active ingredient the compound of the formula

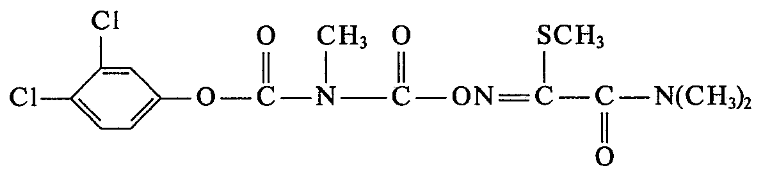

16. A pesticidal composition according to claim 5, which contains as active ingredient the compound of the formula

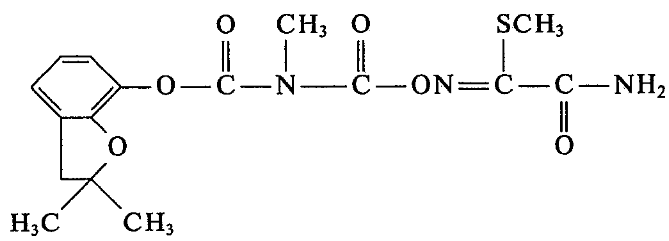

17. A process for the preparation of a carboxylated carbamate of the formula

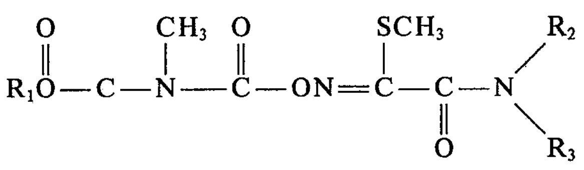

(I)

wherein $R_1$ is a phenyl, naphthyl or dihydrobenzofuranyl radical which is unsubstituted or substituted by halogen atoms, dioxolanyl, dioxanyl, alkyl, alkenyl, alkynyl, alkoxy or alkylthio groups and each of $R_2$ and $R_3$ independently is hydrogen or $C_1$—$C_4$ alkyl, which process comprises reacting a compound of the formula

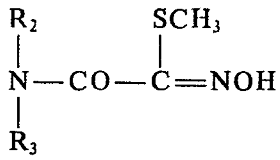

with a compound of the formula

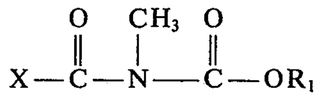

in which formulae $R_1$, $R_2$ and $R_3$ are as defined above and X is a halogen atom, in the presence of a base.

**Revendications pour les Etats contractants: DE, GB, FR, CH, LI, IT, NL, BE**

1. Un carbamate d'oxime carboxylé de formule

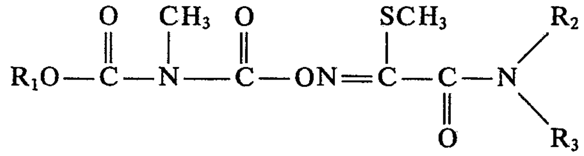

(I)

dans laquelle $R_1$ représente un reste phényle, naphthyle ou dihydrobenzofurannyle éventuellement substitué par des atoms d'halogènes, des groupes dioxolannyle, dioxannyle, alkyle, alcényle, alcynyle, alcoxy ou alkylthio et $R_2$ et $R_3$ représentent chacun, indépendamment l'un de l'autre, l'hydrogène ou un groupe alkyle en C 1—C 4.

2. Un composé selon la rev. 1, dans lequel $R_2$ et $R_3$ représentant chacun, indépendamment l'un de l'autre, l'hydrogène ou un groupe méthyle.

3. Un composé selon l'une des rev. 1 ou 2, dans lequel $R_2$ et $R_3$ représentent chacun un groupe méthyle.

4. Un composé selon l'une des rev. 1 à 3, dans lequel $R_1$ représente un groupe phényle, naphthyle ou dihydrobenzofurannyle non substitué ou un groupe phényle, naphthyle ou dihydrobenzofurannyle substitué par 1 à 3 atomes de fluor, de chlore, de brome ou d'iode ou groupes dioxolannyle, dioxannyle, alkyle, alcényle, alcynyle, alcoxy ou alkylthio.

5. Un composé selon l'une des rev. 2 à 4, dans lequel $R_1$ représente un groupe phényle, 4-chlorophényle, 2-(1-méthoxyméthyl-éthoxy)-phényle, 2-isopropoxyphényle, 2-(1-méthoxy-2-chloréthoxy)-phényle, 3-trifluorométhylphényle, 3,4-dichlorophényle, naphthyle ou 2,2-diméthyl-2,3-dihydrobenzofurannyle.

6. Un composé selon l'une des rev. 3 à 5, dans lequel $R_1$ représente un groupe phényle, 4-chlorophényle, naphthyle ou 2,2-diméthyl-2,3-dihydrobenzofurannyle.

7. Le composé selon la rev. 6, de formule

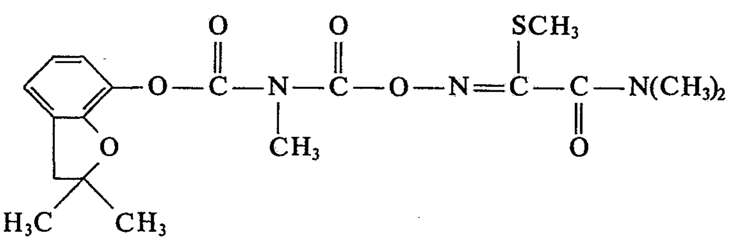

8. Le composé selon la rev. 6, de formule

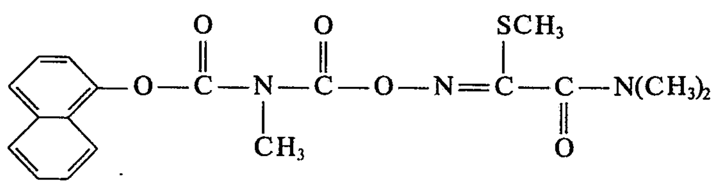

9. Le composé selon la rev. 6, de formule

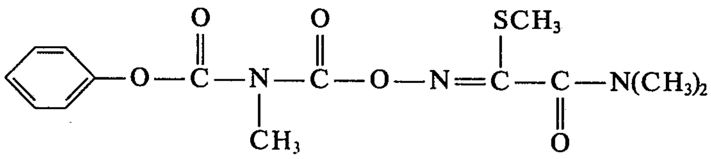

10. Le composé selon la rev. 6, de formule

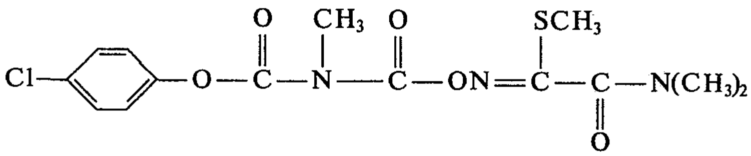

11. Le composé selon la rev. 5, de formule

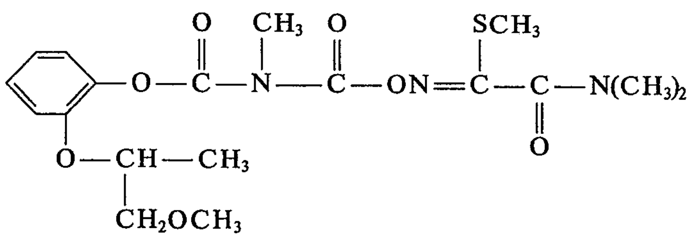

12. Le composé selon la rev. 5, de formule

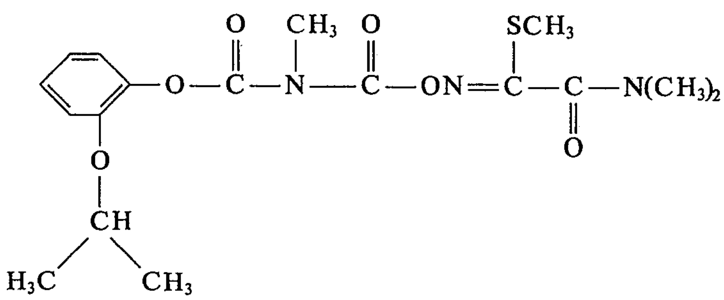

13. Le composé selon la rev. 5, de formule

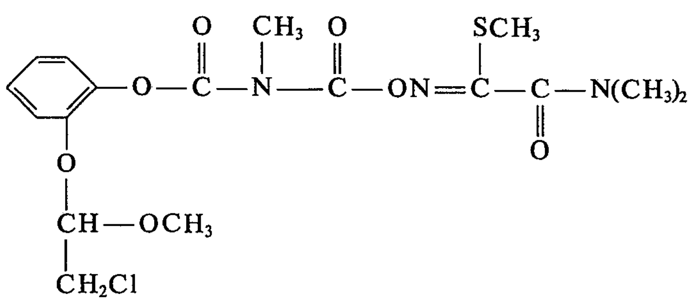

14. Le composé selon la rev. 5, de formule

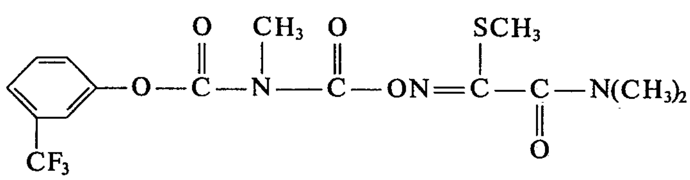

15 Le composé selon la rev. 5, de formule

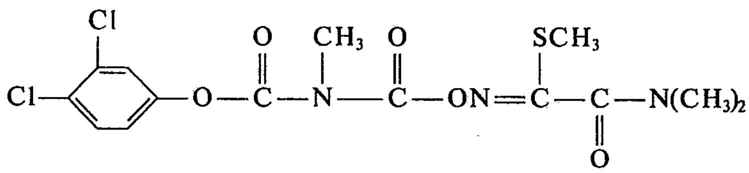

16. Le composé selon la rev. 5, de formule

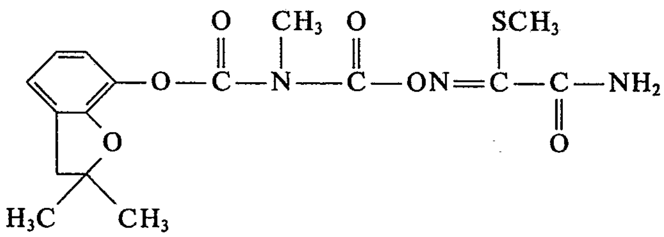

17. Procédé de préparation des composés selon la rev. 1, caractérisé en ce que l'on fait réagir en présence d'une base un composé de formule

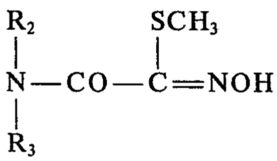

avec un composé de formule

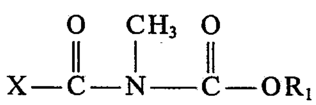

dans lesquelles $R_1$, $R_2$ et $R_3$ ont les significations indiquées dans la rev. 1 et X représente un atome d'halogène.

18. Un produit pesticide contenant en tant que composant actif un composé selon la rev. 1.

19. L'utilisation d'un composé selon la rev. 1, pour combattre les parasites des animaux, des végétaux et du sol.

20. L'utilisation selon la rev. 19, pour combattre les insectes et les représentants de l'ordre des acariens.

**Revendications pour l'Etat contractant: AT**

1. Produit pesticide caractérisé en ce qu'il contient en tant que composant actif, avec des véhicules et/ou d'autres additifs appropriés, un carbamate d'oxime carboxylé de formule

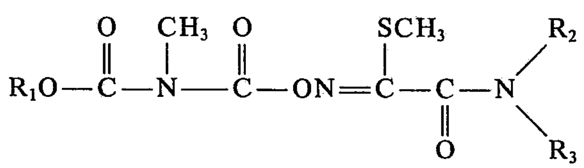

(I)

dans laquelle $R_1$ représente un reste phényle, naphthyle ou dihydrobenzofurannyle éventuellement substitué par des atomes d'halogènes, des groupes dioxolannyle, dioxannyle, alkyle, alcényle, alcynyle, alcoxy ou alkylthio et $R_2$ et $R_3$ représentent indépendamment l'un de l'autre un atome d'hydrogène ou un groupe alkyle en C 1—C 4.

2. Produit pesticide selon la revendication 1, caractérisé en ce qu'il contient en tant que composant actif un composé de formule I dans laquelle $R_2$ et $R_3$ représentent, indépendamment l'un de l'autre, l'hydrogène ou un groupe méthyle.

3. Produit pesticide selon l'une des revendications 1 ou 2, caractérisé en ce qu'il contient en tant que composant actif un composé de formule I dans laquelle $R_2$ et $R_3$ représentent chacun, indépendamment l'un de l'autre, un groupe méthyle.

4. Produit pesticide selon l'une des revendications 1 à 3, caractérisé en ce qu'il contient en tant que composant actif un composé de formule I dans laquelle $R_1$ représente un groupe phényle, naphthyle ou dihydrobenzofurannyle non substitué ou un groupe phényle, naphthyle ou dihydrobenzofurannyle substitué par 1 à 3 atomes de fluor, de chlore, de brome ou d'iode ou groupes dioxolannyle, dioxannyle, alkyle, alcényle, alcynyle, alcoxy ou alkylthio.

5. Produit pesticide selon l'une des revendications 2 à 4, caractérisé en ce qu'il contient en tant que composant actif un composé de formule I dans laquelle $R_1$ représente un groupe phényle, 4-chlorophényle, 2-(1-méthoxyméthyl-éthoxy)-phényle, 2-isopropoxyphényle, 2-(1-méthoxy-2-chloréthoxy)-phényle, 3-trifluorométhylphényle, 3,4-dichlorophényle, naphthyle ou 2,2-diméthyl-2,3-dihydrobenzofurannyle.

6. Produit pesticide selon l'une des revendications 3 à 5, caractérisé en ce qu'il contient en tant que composant actif un composé de formule I dans laquelle $R_1$ représente un groupe phényle, 4-chlorophényle, naphthyle ou 2,2-diméthyl-2,3-dihydrobenzofurannyle.

7. Produit pesticide selon la revendication 6, caractérisé en ce qu'il contient en tant que composant actif le composé de formule

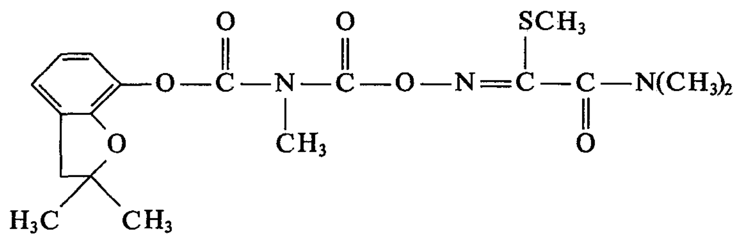

8. Produit pesticide selon la revendication 6, caractérisé en ce qu'il contient en tant que composant actif le composé de formule

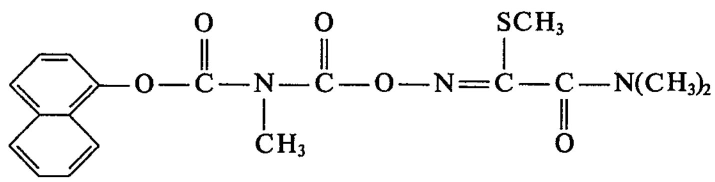

9. Produit pesticide selon la revendication 6, caractérisé en ce qu'il contient en tant que composant actif le composé de formule

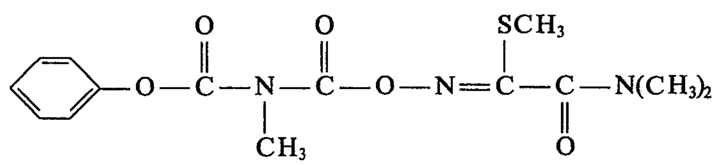

10. Produit pesticide selon la revendication 6, caractérisé en ce qu'il contient en tant que composant actif le composé de formule

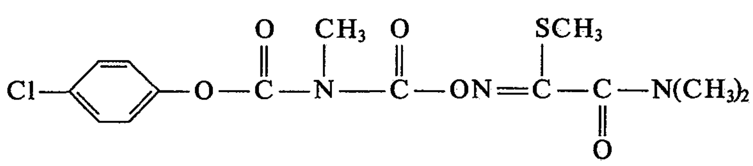

11. Produit pesticide selon la revendication 5, caractérisé en ce qu'il contient en tant que composant actif le composé de formule

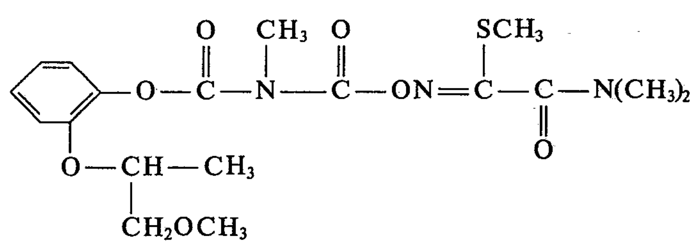

12. Produit pesticide selon la revendication 5, caractérisé en ce qu'il contient en tant que composant actif le composé de formule

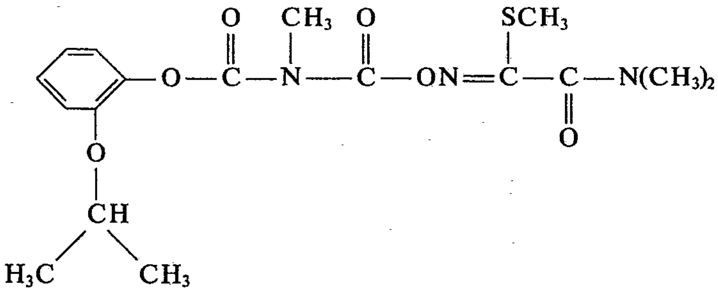

13. Produit pesticide selon la revendication 5, caractérisé en ce qu'il contient en tant que composant actif le composé de formule

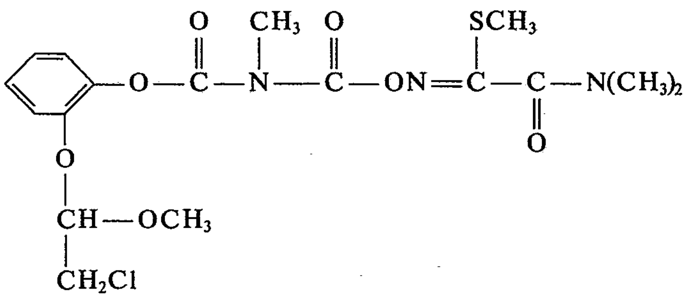

14. Produit pesticide selon la revendication 5, caractérisé en ce qu'il contient en tant que composant actif le composé de formule

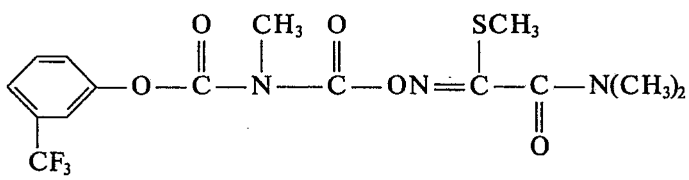

15. Produit pesticide selon la revendication 5, caractérisé en ce qu'il contient en tant que composant actif le composé de formule

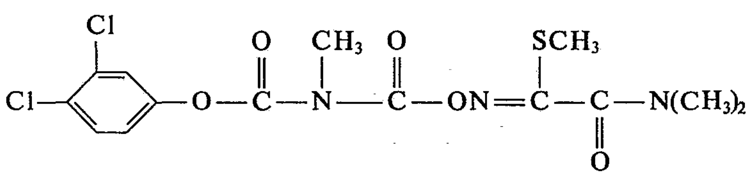

16. Produit pesticide selon la revendication 5, caractérisé en ce qu'il contient en tant que composant actif le composé de formule

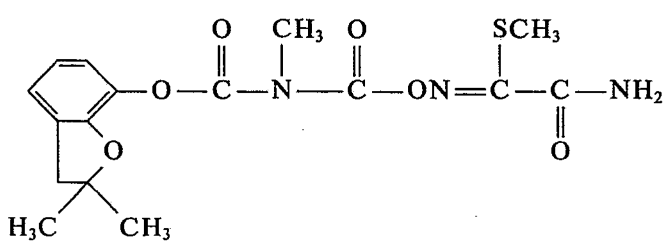

17. Procédé de préparation de carbamates carboxylés de formule

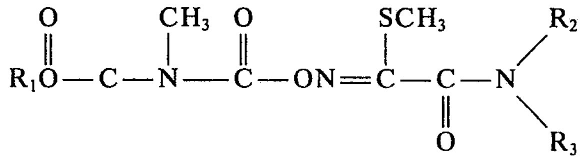 (I)

dans laquelle $R_1$ représente un reste phényle, naphthyle ou dihydrobenzofurannyle éventuellement substitué par des atomes d'halogènes, des groupes dioxolannyle, dioxannyle, alkyle, alcényle, alcynyle, alcoxy ou alkylthio et $R_2$ et $R_3$ représentent chacun, indépendamment l'un de l'autre, l'hydrogène ou un groupe alkyle en C 1—C 4, caractérisé en ce que l'on fait réagir en présence d'une base un composé de formule

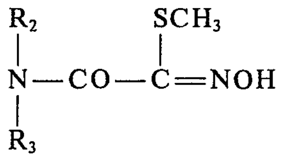

avec un composé de formule

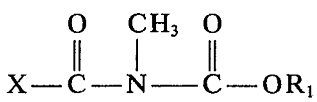

dans lesquelles $R_1$, $R_2$ et $R_3$ ont les significations indiquées ci-dessus et X représente un atome d'halogène.